# EUROPEAN PATENT SPECIFICATION

(11) **EP 2 628 471 B1**
(45) Date of publication and mention of the grant of the patent: **26.07.2017**
(21) Application number: 13445001.4
(22) Date of filing: 15.02.2013
(51) Int. Cl.: A61F 5/441

(54) **Valve**
Ventil
Valve

(30) Priority: 17.02.2012 SE 1200098
(43) Date of publication of application: 21.08.2013
(73) Proprietor: Pysut AB, 59553 Mjölby (SE)
(72) Inventor: Karlsson, Karl-Erik, 58739 Linköping (SE)
(74) Representative: Awapatent AB

(56) References cited:
- GB-A- 1 595 906
- GB-A- 2 270 265
- US-A- 2 524 750

## Description

This invention concerns colostomy bags. Colostomy bags are used by persons that have had a colostomy operation, that is the excrement is passed out through a bowel opening in the abdomen instead of through the rectum. The colostomy bag is provided with a self adhesive plate to fix it against the abdomen. In the adhesive plate there is an opening that is adopted to the bowel (stomia). Colostomy bags are provided with filter -provided openings in the upper part for letting out the air/gas that is naturally formed in the bowels and end up in the bag.

From the technical field of valves in connection with colostomy bags US5,524,750 can be mentioned. However here the object of the valve is to let air enter as opposed to the invention. Also GB 1 595 906 and GB 2 270 265 can be mentioned which are concerned with the removal of gas. Both however deals with the problem to be solved in different way namely with a filter after valve whereas the invention has as its object to prevent contaminations to enter the valve. A large problem with colostomy bags is that when the user is lying down the filters become more or less clogged with excrement. Then they do not let out the air with the required speed and the bags often get blown up. It is very uncomfortable to walk with an inflated bag on the abdomen , which also considerably increase the risk of the bag coming loose from the abdomen.

The object of the invention is to solve the above problem. This is in accordance with claim 1 achieved by an easily maneuverable valve in the upper end of the bag. When the valve is opened the surplus air is released. The valve has a cleaning function on the inside that prevents excrement to accompany the surplus air out and block the opening. The cleaning function is of central importance. Through its simple technical design the function of the valve is secured. This is a very important feature of the valve described here.

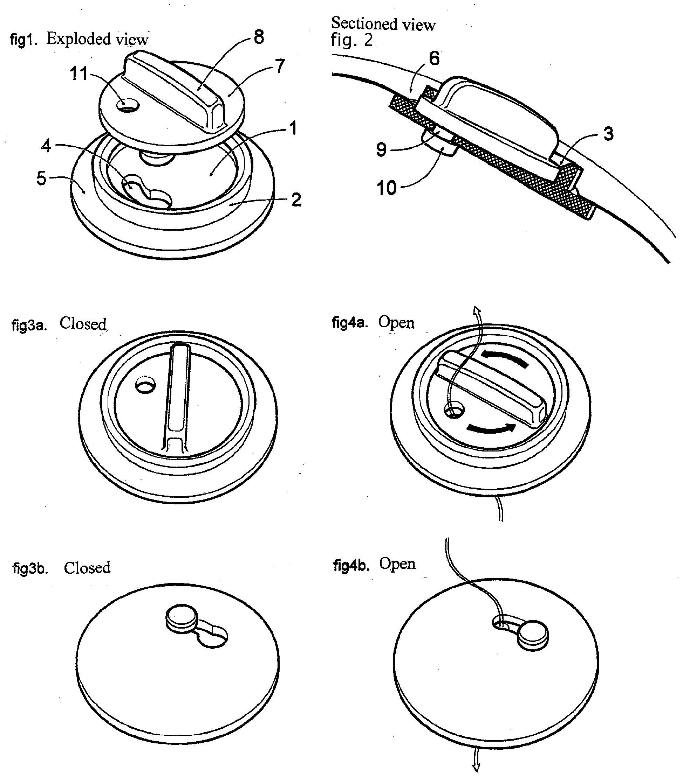

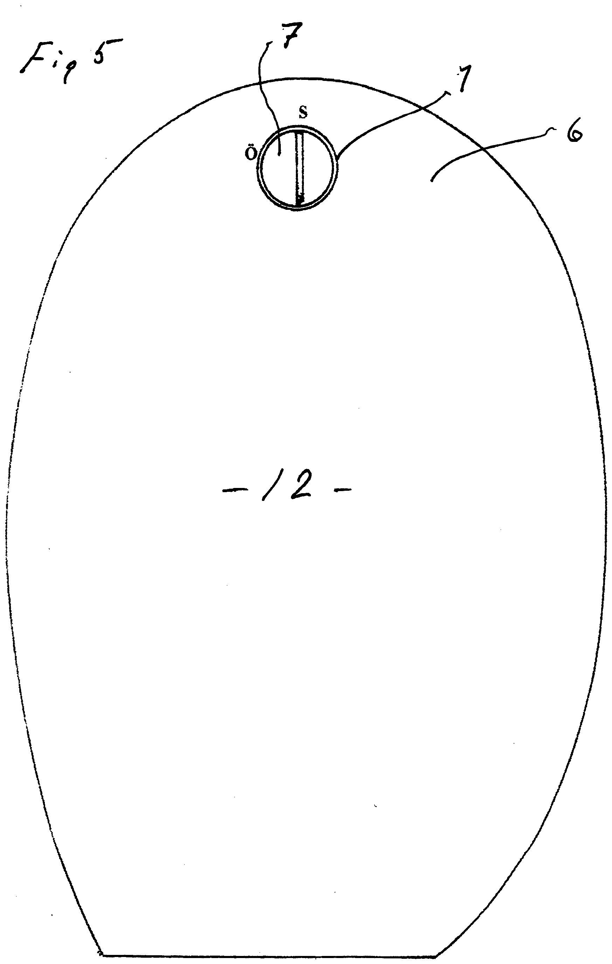

An embodiment of a valve in accordance with the invention is in the following described in greater detail with reference to the enclosed drawing where fig. 1 depicts a an exploded view of the valve, fig. 2 depicts the valve partly in section, Fig 3a show the valve seen from the outside in closed position, fig 3b the valve in closed position seen from the inside, fig 4a the valve in open position seen from the outside, fig 4b show the valve seen from the inside in open position and fig 5 a valve-provided colostomy bag.

The valve shown in the drawing comprise, a valve body A that surround a turn able inner swivel slide B.

### A. Valve body.

The valve body 1 is made of a plastic that can be welded to an inner polyethene bag of the colostomy bag. It has on the outside a circular concentrically placed space limited by a rim 2 that on the upper edge on the inside has a conical and slightly inwards protruding flange 3 that is chamfered towards the inner periphery, this for facilitating the following assembly. The valve body has in the bottom a curved "keyhole shaped" opening 4. This opening is in the following named "opening in the valve body". On the outside of the rim there is a circle shaped area 5 dedicated to a weld joint to a circular opening in the upper end 6 of the colostomy bag.

### B. Swivel slide

The swivel slide 7 is made of a rigid plastic quality. It's outer dimensions shall with such a fit be accommodated within the limits of the rim and the flange that so that no leakage will occur. On its upper side it has a handle/grip 8. On the bottom side there is a pin 9 with a collar (widening) 10. The length of the pin between the bottom of the swivel slide and the collar shall be the same as the thickness of the bottom of the valve body bottom. The swivel slide is provided with a venting hole 11. Both parts shall have an even surface to provide a good seal against each other. At the assembly of the valve parts the swivel slide is pressed inside the rim and is retained there by the flange 3. The collar of the pin shall at assembly pass through the larger opening in the valve body opening.

### Venting of colostomy bag

When air is to be let out from the bag by means of the valve the body is to be in an upright position. Before the valve is opened the user shall have pressed lightly from above and down on the upper end of the bag so that there are no larger amounts of excrement. The swivel slide is turned towards the open position and the pin with the collar set free the area on the back side of the valve body, where the venting hole of the swivel slide will be when the valve is fully open. During the turning movement the pin push away excrement that has ended up in the part of the valve body opening that has not been shielded by the pin and the collar. The collar leaves a clean area along the edge of the valve body opening 4. The collar thus has a very important function to prevent the venting hole from being blocked by excrement. The turning is continued until the pin abuts the other end of the opening in the valve body. The valve is then open and with a light pressure on the bag the trapped air can flow out. Thereafter the swivel slide only has to be re-turned and the valve is again closed.

Practical tests have shown that only air comes out, this is in the top of the bag and as long as the bag I not fully expanded and fully filled with excrements there will in at the top be a space with air.

By making the valve parts of for instance plastic of appropriate quality and with good mutual fit for the two parts a good seal can be achieved. Pin and opening can if so is desired be shaped so that the closed position has a snap lock.

The valve can instead of being maneuvered by turning be a linear push slide with a corresponding basic function so that the opening in the valve body and its closest surrounding is covered when the valve is closed. It must be clean around the opening in the valve body when the valve is opened.

Since the collar on the pin when the valve is closed may come in contact with the excrement the opening in the valve body should be sufficiently long for the air flow velocity there to be so small there that no excrement is pulled along. At the opening in the valve body there may furthermore or instead be a stop flange that the collar abut in closed position.

## Claims

1. Venting valve for colostomy bags, comprising a valve body (1) in which a slide (7) is turnable in order to bring a venting hole (11) in the slide to agreement with an opening (4) in the valve body, comprising a pin or heel (9) on the face of the slide facing the valve body
**characterized in that**
the opening in the valve body is extended in the movement direction of the slide and
the pin or heel extends into the opening in the valve body, said pin or heel (9) having a shorter length in the movement direction of the slide than the opening in the valve body so that the slide can be moved between an open and a closed position, the opening in the valve body having an end area that is set free by the slide, the pin or the heel (9) having a shape and dimension corresponding to the shape and width of the end area for sealing contact in the closed position, and the pin or heel (9) in its free end is provided with a laterally protruding collar (10) or extension that on the side of the valve body facing away from the slide (7) is in contact with the valve body and when the valve is closed extend a distance outside the opening (4) in the valve body so that also an area around the air passage is kept free from contaminations.

2. Venting valve according to claim 1, **characterized in that** the valve body at a distance from the intended air passage has a widening of the elongate opening so that valve body and valve slide can easily be assembled.

3. Venting valve according to claim 2 **characterized in that** valve slide and pin or heel are in one piece.

4. Venting valve according to any of the preceding claims **characterized in that** the length of the opening (4) in the valve body is longer than what is required for the opening in the valve body and the venting hole in the slide to correspond.

5. Venting valve according to any of the preceding claims, **characterized in that** on the inside of the valve body is a heel or contact flange arranged, that when the valve is closed abut the collar of the pin on the side facing away from the opening in the valve body.

## Patentansprüche

1. Entlüftungsventil für Kolostomiebeutel, umfassend einen Ventilkörper (1), in dem ein Schieber (7) drehbar ist, um ein Entlüftungsloch (11) in dem Schieber mit einer Öffnung (4) in dem Ventilkörper in Übereinstimmung zu bringen, umfassend einen Stift oder Absatz (9) auf der Fläche des Schiebers, der zum Ventilkörper weist,
**dadurch gekennzeichnet, dass**
sich die Öffnung in dem Ventilkörper in der Bewegungsrichtung des Schiebers erstreckt und sich der Stift oder Absatz in die Öffnung in dem Ventilkörper erstreckt, der Stift oder Absatz (9) eine kürzere Länge in der Bewegungsrichtung des Schiebers als die Öffnung in dem Ventilkörper aufweist, sodass der Schieber zwischen einer offenen und einer geschlossenen Position bewegt werden kann, die Öffnung in dem Ventilkörper einen Endbereich aufweist, der von dem Schieber freigegeben wird, der Stift oder der Absatz (9) eine Form und Abmessung aufweist, die der Form und Breite des Endbereichs für einen abdichtenden Kontakt in der geschlossenen Position entsprechen, und der Stift oder Absatz (9) in seinem freien Ende mit einem lateral hervorstehenden Kragen (10) oder einer Verlängerung versehen ist, die auf der Seite des Ventilkörpers, die weg von dem Schieber (7) weist, in Kontakt mit dem Ventilkörper steht, und wenn das Ventil geschlossen ist, sich um einen Abstand außerhalb der Öffnung (4) in dem Ventilkörper erstreckt, sodass auch ein Bereich um den Luftkanal frei von Kontaminationen gehalten wird.

2. Entlüftungsventil nach Anspruch 1, **dadurch gekennzeichnet, dass** der Ventilkörper um einen Abstand von dem vorgesehenen Luftkanal eine Aufweitung der länglichen Öffnung aufweist, sodass der Ventilkörper und der Ventilschieber leicht zusammengebaut werden können.

3. Entlüftungsventil nach Anspruch 2, **dadurch gekennzeichnet, dass** der Ventilschieber und der Stift oder Absatz einstückig ausgebildet sind.

4. Entlüftungsventil nach einem der vorhergehenden Ansprüche, **dadurch gekennzeichnet, dass** die Länge der Öffnung (4) in dem Ventilkörper länger ist, als für die Übereinstimmung der Öffnung in dem Ventilkörper und des Entlüftungslochs in dem Schieber erforderlich ist.

5. Entlüftungsventil nach einem der vorhergehenden Ansprüche, **dadurch gekennzeichnet, dass** auf der Innenseite des Ventilkörpers ein Absatz oder Kontaktflansch angeordnet ist, der, wenn das Ventil geschlossen ist, an den Kragen des Stiftes auf der Seite angrenzt, die weg von der Öffnung in dem Ventilkörper weist.

## Revendications

1. Valve de ventilation pour des sachets de colostomie, comprenant un corps de valve (1) dans lequel un volet mobile (7) peut être tourné pour amener un trou de ventilation (11) dans l'obturation en correspondance avec une ouverture (4) dans le corps de valve, comprenant une tige ou un talon (9) sur la face du volet mobile face au corps de valve,
**caractérisée en ce que**
l'ouverture dans le corps de valve est étendue dans la direction de mouvement du volet mobile et la tige ou le talon s'étend jusque dans l'ouverture dans le corps de valve, ladite tige ou ledit talon (9) présentant une longueur plus courte dans la direction de mouvement du volet mobile que l'ouverture dans le corps de valve de façon à ce que le volet mobile puisse être déplacé entre une position ouverte et une position fermée, l'ouverture dans le corps de valve ayant une zone d'extrémité qui est libérée par le volet mobile, la tige ou le talon (9) ayant une forme et une dimension correspondant à la forme et à la largeur de la zone d'extrémité pour un contact étanche dans la position fermée, et la tige ou le talon (9) dans son extrémité libre est doté(e) d'un collier (10) en saillie ou en extension radiale qui, sur le côté du corps de valve orienté en s'éloignant du volet mobile (7), est en contact avec le corps de valve et lorsque la valve est fermée, s'étend d'une distance hors de l'ouverture (4) dans le corps de valve de façon à ce qu'une zone autour du passage d'air soit maintenue exempte de contaminations.

2. Valve de ventilation selon la revendication 1, **caractérisée en ce que** le corps de valve à une distance du passage d'air prévu présente un élargissement de l'ouverture allongée de façon à ce que le corps de valve et le volet mobile de valve puissent être assemblés facilement.

3. Valve de ventilation selon la revendication 2, **caractérisée en ce que** le volet mobile de valve et la tige ou le talon sont d'une seule pièce.

4. Valve de ventilation selon l'une des revendications précédentes, **caractérisée en ce que** la longueur de l'ouverture (4) dans le corps de valve est plus longue que ce qui est requis pour que l'ouverture dans le corps de valve et le trou de ventilation dans le volet mobile correspondent.

5. Valve de ventilation selon l'une des revendications précédentes, **caractérisée en ce qu'**à l'intérieur du corps de valve, il y a un talon ou une bride de contact agencé(e), qui lorsque la valve est fermée, est adjacent(e) au collier de la tige sur le côté orienté en s'éloignant de l'ouverture dans le corps de soupape.
